# EUROPEAN PATENT APPLICATION

(11) **EP 3 679 852 A1**
(43) Date of publication of application: **15.07.2020**
(21) Application number: 18854146.0
(22) Date of filing: 31.08.2018
(51) Int. Cl.: A61B 1/04, A61B 1/00, A61B 8/08

(54) **ENDOSCOPIC DEVICE FOR ORAL USE**

(30) Priority: 05.09.2017 KR 20170113136
(71) Applicant: Endolfin Co., Ltd., Gyeonggi-do 14056 (KR)
(72) Inventor: SHIM, Han Bo, Seongnam-si, Gyeonggi-do 13597 (KR)
(74) Representative: Caspary, Karsten
(86) International application number: PCT/KR2018/010111
(87) International publication number: WO 2019/050218

(57) **Abstract**

Disclosed is a peroral endoscopic device capable of being swallowed, including: one or more imaging units performing imaging for the digestive organs and outputting image data for the digestive organs; one or more ultrasonic units outputting ultrasound data for the submucosal regions beneath the inner walls of the digestive organs; a magnetic unit adjusting a position, a posture and an advancing direction of the peroral endoscopic device in response to an external magnetic force; a transceiving unit transmitting the image data and the ultrasound data to an external device or receiving an external control signal; a control unit controlling the imaging units and the ultrasonic units so as to perform the imaging for the digestive organs and the submucosal regions; and a power unit supplying power to the imaging units, the ultrasonic units, the magnetic unit, the transceiving unit, and the control unit.

## Description

### BACKGROUND OF THE INVENTION

### Cross Reference to Related Application of the Invention

The present application claims the benefit of Korean Patent Application No.10-2017-0113136 filed in the Korean Intellectual Property Office on September 5, 2017, the entire contents of which are incorporated herein by reference.

### Field of the Invention

The present invention relates to a peroral endoscopic device, and more particularly, to a peroral endoscopic device that is capable of monitoring digestive organs as well as the surrounding organs around the digestive organs.

### Background of the Related Art

The digestive organs of the human body are largely divided into the esophagus, stomach, small intestine, and large intestine. Typically, the esophagus and the stomach are observed by an upper gastrointestinal endoscopy that can be inserted up to the distal portion of the duodenum, and the large intestine is observed by a colonoscopy that can be inserted up to the terminal ileum as the end portion of the small intestine.

However, an endoscopy capable of diagnosing and treating the small intestine has been not suggested until now. In conventional practices, accordingly, various radiation diagnosis inclusive of a barium swallow and CT imaging have been applied to the small intestine, but in this case, unfortunately, a diagnosis rate for diseases of the small intestine is substantially low.

So as to solve such conventional problems, recently, many studies and developments for a peroral endoscopy have been made. The peroral endoscopy is a capsule type endoscopy that is capable of being swallowed and performing the close up imaging for the esophagus, stomach, small intestine, and large intestine by means of a tiny camera.

However, it is hard to stop the existing peroral endoscopy at a portion where the digestive organ is abnormal or a precise observation is needed or to move the existing peroral endoscopy to the already passing portion of the digestive organ so as to observe the portion again. This is because the existing peroral endoscopy is not moved by means of its own force, but it is moved passively by means of the peristaltic motion of the digestive organ.

Also, some of peroral endoscopic systems control the position and posture of the peroral endoscopy in the stomach on the outside by means of a magnetic device providing a strong magnetic field around the human body, but in this case, the control device is large, so that disadvantageously, the peroral endoscopic system is bulky, the price of the system is high, and the operating cost of the system is also high.

Through ultrasonic sensors mounted on the existing peroral endoscopy, further, studies on the diagnosis for the submucosal and muscularis tumors in the stomach and the surrounding organs like the pancreas around the stomach have been introduced, but as an air layer between the peroral endoscopy and the inner wall of the digestive organ is produced, it is found that it is impossible to perform ultrasonic imaging itself. So as to solve such conventional problems, it is very effective to allow the peroral endoscopy to come into close contact with the inner wall of the digestive organ, but the problems have been not perfectly solved yet. Accordingly, CT imaging or ultrasound imaging using a probe has to be additionally used to diagnose the surrounding organs like the pancreas around the stomach.

### SUMMARY OF THE INVENTION

Accordingly, the present invention has been made in view of the above-mentioned problems occurring in the related art, and it is an object of the present invention to provide a peroral endoscopic device that is capable of being controlled in a posture, moving direction, a moving speed, and an imaging direction, thereby achieving accurate monitoring and diagnosis for various digestive organs such as the relatively large stomach as well as the small and large intestines.

It is another object of the present invention to provide a peroral endoscopic device that is capable of more accurately diagnosing diseases hard to be accurately diagnosed only with an endoscope, through ultrasonic waves.

It is yet another object of the present invention to provide a peroral endoscopic device that is capable of being miniaturized and made at a low cost, so that the peroral endoscopic device can be controlled in movement and imaging direction and also can carefully perform imaging for the inner/outer walls of the digestive organs through ultrasonic waves.

It is still another object of the present invention to provide a peroral endoscopic device that is capable of performing ultrasonic imaging for the surrounding organs around the stomach, without having any existing endoscopic ultrasonography using a probe.

To accomplish the above-mentioned objects, according to the present invention, there is provided a peroral endoscopic device capable of being swallowed, including: one or more imaging units moving to the digestive organs of the human body, performing imaging for the digestive organs, and outputting image data for the digestive organs; one or more ultrasonic units for outputting ultrasound data for the regions (hereinafter, referred to as submucosal regions') beneath the inner walls of the digestive organs and the surrounding organs around the digestive organs; a magnetic unit for adjusting a position, a posture and an advancing direction of the peroral endoscopic device in response to an external magnetic force; a transceiving unit for transmitting the image data and the ultrasound data to an external device or for receiving an external control signal; a control unit for controlling one or more imaging units and one or more ultrasonic units so as to perform the imaging for the digestive organs and the submucosal regions simultaneously or individually; and a power unit for supplying power to one or more imaging units, one or more ultrasonic units, the magnetic unit, the transceiving unit, and the control unit.

According to the present invention, desirably, the external magnetic force is generated from an external magnetic force controller for operating the magnetic unit, and the magnetic force controller comes into close contact with or is close to an upper body of a patient to apply the external magnetic force to the magnetic unit.

According to the present invention, desirably, the magnetic force controller controls the magnetic unit to allow the peroral endoscopic device to be moved to the inner wall of the stomach closest to the surrounding organs, and if the peroral endoscopic device is moved to the inner wall of the stomach closest to the surrounding organs, the control unit controls one or more imaging units to perform the imaging for the inner wall of the stomach closest to the surrounding organs and one or more ultrasonic units to perform the imaging for the submucosal regions of the inner wall of the stomach closest to the surrounding organs.

According to the present invention, desirably, if the surrounding organ is the pancreas, the control unit controls one or more ultrasonic units so that pancreas imaging is performed in a state where a frequency for imaging the center of the pancreas is different from frequencies for imaging the head and tail of the pancreas.

According to the present invention, desirably, an image sensor of one or more imaging units and one or more ultrasonic transducers of one or more ultrasonic units are arranged serially on the same line as the peroral endoscopic device so as to perform the imaging for the inner wall of the digestive organ in the same direction as each other.

According to the present invention, desirably, if the ultrasonic transducers are two or more, the image sensor of one or more imaging units is located between the ultrasonic transducers so as to allow the angle of view of the image sensor to be overlapped with the ultrasonic scanning range of the ultrasonic transducers.

According to the present invention, desirably, the surface of the peroral endoscopic device facing one or more ultrasonic units is curvedly formed to have positive curvature.

According to the present invention, desirably, an image sensor of one or more imaging units and one or more ultrasonic transducers of one or more ultrasonic units are arranged in two rows and parallel with each other along the inner peripheral surface of the peroral endoscopic device so as to perform the imaging for the inner wall of the digestive organ in the same direction as each other.

According to the present invention, desirably, one or more ultrasonic transducers of one or more ultrasonic units are arranged along at least one arc of the peroral endoscopic device as a portion of the circumference thereof.

According to the present invention, desirably, the magnetic unit is adapted to allow the peroral endoscopic device to come into close contact with the inner wall of the digestive organ through the interaction with the external magnetic force.

According to the present invention, desirably, the power unit includes at least one of a chargeable battery and a general battery.

According to the present invention, desirably, one or more ultrasonic units come into close contact with the surface of the peroral endoscopic device that faces the ultrasonic units, and the surface of the peroral endoscopic device facing one or more ultrasonic units is flattened.

According to the present invention, desirably, the magnetic unit includes: a first permanent magnet; and a second permanent magnet, and the power unit as a conductor is located between the first permanent magnet and the second permanent magnet.

### BRIEF DESCRIPTION OF THE DRAWINGS

The above and other objects, features and advantages of the present invention will be apparent from the following detailed description of the embodiments of the invention in conjunction with the accompanying drawings, in which:
FIGS.1 to 4 are block diagrams showing peroral endoscopic systems according to first to fourth embodiments of the present invention, to which peroral endoscopic devices capable of being swallowed are applied;
FIG.5 is a block diagram showing a peroral endoscopic device according to the present invention;
FIG.6 is a block diagram showing an ultrasonic unit of the peroral endoscopic device according to the present invention;
FIG.7 is a schematic view showing the pancreas located around the stomach of the digestive organs of the human body;
FIG.8 is a concept view showing an example of an arrangement of two permanent magnets and the power unit in the peroral endoscopic device according to the present invention;
FIGS.9 to 12B are schematic views showing first to fifth peroral endoscopic devices according to first to fifth embodiments of the present invention; and
FIG.13 is a flowchart showing an endoscopic method for the pancreas through the peroral endoscopic device according to the present invention.

### DETAILED DESCRIPTION OF THE EMBODIMENTS

Objects, characteristics and advantages of the present invention will be more clearly understood from the detailed description as will be described below and the attached drawings. Before the present invention is disclosed and described, it is to be understood that the disclosed embodiments are merely exemplary of the invention, which can be embodied in various forms. Therefore, specific structural and functional details disclosed herein are not to be interpreted as limiting, but merely as a basis for the claims and as a representative basis for teaching one of ordinary skill in the art to variously employ the present invention in virtually any appropriately detailed structure.

In the description, when it is said that one member is located "above" or "under" another member, it means that one member may come into contact with another member as well as yet another member may exist between the two members. In the description, also, the thicknesses of the lines or the sizes of the components shown in the drawing may be magnified for the clarity and convenience of the description.

Terms, such as the first, and the second may be used to describe various elements, but the elements should not be restricted by the terms. The terms are used to only distinguish one element from the other element.

Also, when a first element is operated or implemented on a second element, it should be understood that the first element is operated or implemented on an environment where the second element is operated or implemented, and otherwise, the first element is operated or implemented through direct or indirect interactive operations with the second element.

If an element, component, device, or system includes a part constituted of a program or software, it should be understood that the element, component, device, or system includes hardware (e.g., memory, CPU, etc.) or other programs or software (e.g., a driver for driving an operating system or hardware, etc.) as required to operate or implement the program or software unless otherwise noted.

Unless specially described otherwise in implementing an element, it should be understood that the element can be implemented in the form of software, hardware, or any one of the software and hardware.

Terms used in this application are used to only describe specific exemplary embodiments and are not intended to restrict the present invention. An expression referencing a singular value additionally refers to a corresponding expression of the plural number, unless explicitly limited otherwise by the context. When it is said that one portion is described as "includes" any component, one element further may include other components unless no specific description is suggested.

Hereinafter, the present invention will be in detail explained with reference to the attached drawings. The present invention may be modified in various ways and may have several exemplary embodiments. Specific exemplary embodiments of the present invention are illustrated in the drawings and described in detail in the detailed description. However, this does not limit the invention within specific embodiments and it should be understood that the invention covers all the modifications, equivalents, and replacements within the idea and technical scope of the invention.

If it is determined that the detailed explanation on the well known technology related to the present invention makes the scope of the present invention not clear, the explanation will be avoided for the brevity of the description.

The term "module" described in the specification indicates a functional or structural combination of the hardware for implementing the technical scope of the present invention and the software for driving the hardware. For example, the module means a logical unit of given codes and a hardware resource through with the given codes are executed, and it can be easily understood by those having the ordinary skill in the art that the codes are not necessarily connected physically and the hardware does not mean one kind of hardware.

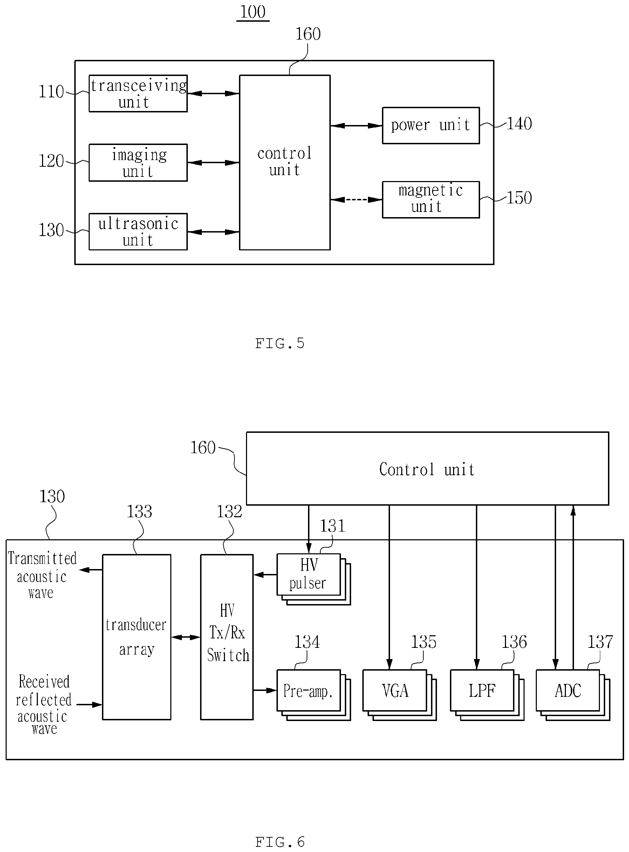

Further, first to fifth peroral endoscopic devices 100, 600 to 900 and 900a according to the present invention, which are capable of being swallowed as will be explained with reference to FIGS.5, 6, and 9 to 12B, are configured to have their units separated functionally and logically from each other, and it can be easily understood by those having the ordinary skill in the art that the units are distinguished by separate physical devices or made with separate codes.

Hereinafter, the present invention will be in detail explained with reference to the attached drawings.

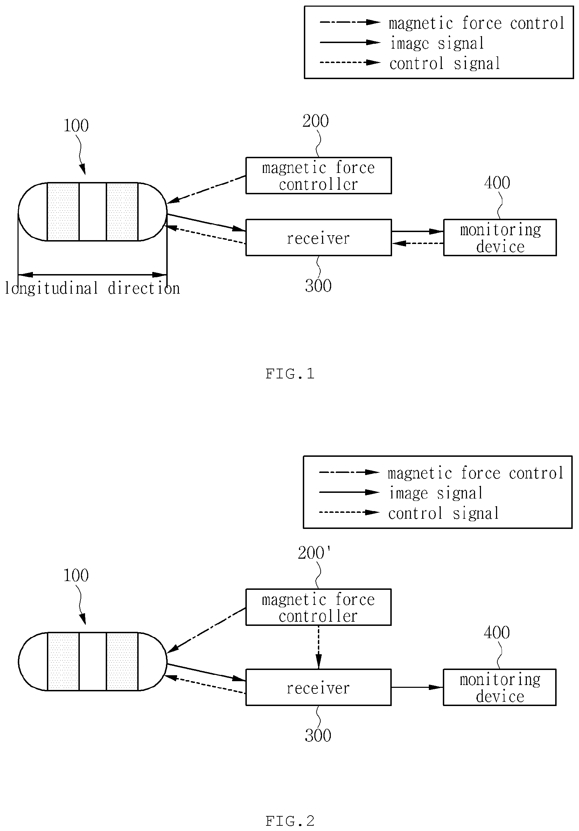

FIG.1 is a schematic block diagram showing a peroral endoscopic system according to a first embodiment of the present invention, to which a peroral endoscopic device capable of being swallowed is applied.

As shown in FIG.1, the peroral endoscopic system according to the first embodiment of the present invention includes the peroral endoscopic device 100, a magnetic force controller 200, a receiver 300, and a monitoring device 400.

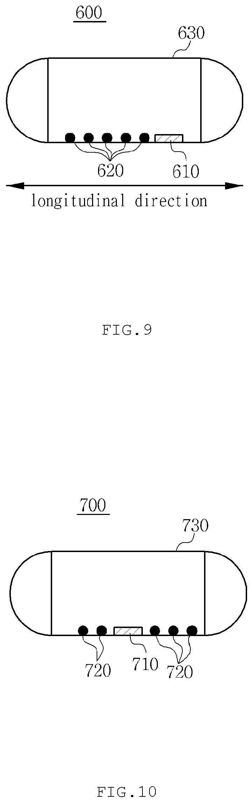

The peroral endoscopic device 100 as shown in FIG.1 may be one of the first to fifth endoscopic devices 600, 700, 800, 900 and 900a as will be discussed later with reference to FIGS.9 to 12B.

The peroral endoscopic device 100 has the form of a capsule capable of being swallowed and can perform imaging through an image sensor and ultrasound imaging through an ultrasonic sensor. Further, the peroral endoscopic device 100 according to the present invention includes permanent magnets, and also, it can be controlled in a posture and an advancing direction through an external magnetic force generated from the magnetic force controller 200. Through the control of the posture, particularly, the peroral endoscopic device 100 comes into close contact with the inner wall of the stomach by means of the permanent magnets and the magnetic force controller 200 so that it can perform imaging for the inner wall of the stomach or the submucosal region beneath the inner wall of the stomach.

The magnetic force controller 200 makes use of an external magnet for generating the external magnetic force to control the position, posture and advancing direction of the peroral endoscopic device 100, and according to the present invention, the magnetic force controller 200 may be a handheld type device capable of being held by an operator's hand or be mounted on an end of an arm of an articulated robot in such a manner as to be automatically or semi-automatically controlled remotely. In this case, it is important that the magnetic force controller 200 has a small size capable of being controlled by the hand or the robot arm so that it can come into close contact with or be close to the upper body of a patient to directly apply the magnetic force to the peroral endoscopic device 100. This is clearly different from a large-sized magnetic device for producing a magnetic field around the human body in the conventional practice.

The magnetic force controller 200 is located near the abdominal region of the patient who swallows the peroral endoscopic device 100 by means of the control of the operator, and next, the intensities of the magnetic force can be adjusted adequately to the body state of the patient by means of the control of the operator. In detail, the operator places the magnetic force controller 200 on the abdominal region of the patient who swallows the peroral endoscopic device 100 and then checks the position of the peroral endoscopic device 100 in the interiors of the digestive organs of the patient through the images of the monitoring device 400.

If the magnetic force controller 200 is moved on the abdominal region of the patient by means of the control of the operator, further, the direction of the magnetic force generated from the external magnet is varied to control the posture or advancing direction of the peroral endoscopic device 100. In detail, the magnetic force controller 200 can control the posture or advancing direction of the peroral endoscopic device 100 through the operator's manual operation (or remote control of the robot arm), and accordingly, the peroral endoscopic device 100 is moved to the inner wall of the digestive organ, for which imaging is required, to perform the imaging for the inner wall or the submucosal region of the inner wall through the image sensor and/or ultrasound sensor, and to wirelessly transmit the image data or ultrasound data to the receiver 300.

The receiver 300 receives the image data or ultrasound data outputted from the peroral endoscopic device 100 and transmits the received data to the monitoring device 400. Further, the receiver 300 receives operation control signals for controlling operations of the peroral endoscopic device 100 from the monitoring device 400 and transmits the received signals to the peroral endoscopic device 100. For example, the operation control signals include a signal for controlling imaging units 120 and ultrasonic units 130 as will be discussed later so that the imaging units 120 and the ultrasonic units 130 perform the imaging simultaneously or sequentially.

The monitoring device 400 is a personal computer that analyzes the image data and the ultrasound data received from the receiver 300, processes the data in the form capable of being displayed, and displays the processed data on a screen.

FIG.2 is a schematic block diagram showing a peroral endoscopic system according to a second embodiment of the present invention, to which a peroral endoscopic device 100 capable of being swallowed is applied.

As shown in FIG.2, the peroral endoscopic system according to the second embodiment of the present invention is similar to the peroral endoscopic system as explained with reference to FIG.1.

Referring again to FIG.1, the magnetic force controller 200 controls the posture and advancing direction of the peroral endoscopic device 100, and the operation control signals are generated from the monitoring device 400.

Referring to FIG.2, contrarily, a magnetic force controller 200' generates the operation control signals and outputs the operation control signals to the receiver 300. The receiver 300 transmits the operation control signals received from the magnetic force controller 200' to the peroral endoscopic device 100.

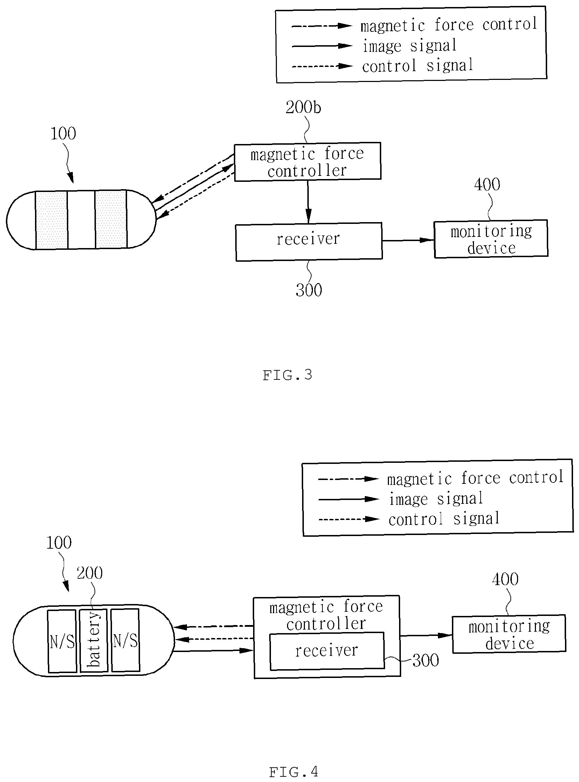

FIG.3 is a schematic block diagram showing a peroral endoscopic system according to a third embodiment of the present invention, to which a peroral endoscopic device 100 capable of being swallowed is applied.

As shown in FIG.3, the peroral endoscopic system according to the third embodiment of the present invention is similar to the peroral endoscopic system as explained with reference to FIG.1.

Referring to FIG.3, however, a magnetic force controller 200b generates the operation control signals for controlling the peroral endoscopic device 100 and directly transmits the operation control signals to the peroral endoscopic device 100. Also, the magnetic force controller 200b transmits the image data or ultrasound data of each sensor received from the peroral endoscopic device 100 to the receiver 300. In this case, the peroral endoscopic device 100 can communicate with the magnetic force controller 200b by means of galvanic coupling or RF communication technology as will be discussed later.

FIG.4 is a schematic block diagram showing a peroral endoscopic system according to a fourth embodiment of the present invention, to which a peroral endoscopic device 100 capable of being swallowed is applied.

As shown in FIG.4, the peroral endoscopic system according to the fourth embodiment of the present invention is similar to the peroral endoscopic system as explained with reference to FIG.3. Referring to FIG.4, however, a magnetic force controller 200c has the receiver 300 built therein. The magnetic force controller 200c collects and stores the image data or ultrasound data received from the peroral endoscopic device 100 in the receiver 300 and transmits the stored image data or ultrasound data to the monitoring device 400.

FIG.5 is a block diagram showing the peroral endoscopic device according to the present invention.

Referring to FIG.5, the peroral endoscopic device 100 according to the present invention includes a transceiving unit 110, imaging units 120, ultrasonic units 130, a power unit 140, a magnetic unit 150, and a control unit 160.

The transceiving unit 110 receives the operation control signals outputted from the monitoring device 400 from the receiver 300 or the magnetic force controller 200a, 200b, or 200c and transmits the received signals to the control unit 160. Further, the transceiving unit 110 transmits the image data outputted from the imaging units 120 or the ultrasound data outputted from the ultrasonic units 130 to an external device like the receiver 300.

The transceiving unit 110 makes use of the galvanic coupling or the RF communication technology requiring an antenna and also supports simplex radio communication or duplex radio communication. The transceiving unit 110 is constituted of one or more ASICs (Application Specific Integrated Circuits).

The galvanic coupling is a method for allowing a signal electrode (+) and a grounding electrode (-) of the transceiving unit 110 to be attached to the human body to transmit a signal through changes in an electric field caused by a potential difference between the two electrodes. In this case, advantageously, the galvanic coupling does not need any antenna for transmitting and receiving the signal and provides low power consumption and miniaturization.

One or more imaging units 120 serve to perform imaging for the inner walls of the digestive organs in real time or intermittently during the peroral endoscopic device 100 is moved along the digestive organs of the human body and thus to output the image data in a visible light region. To do this, the imaging units 120 include optical elements for camera imaging, such as one or more image sensors like CCD (Charge Coupled Device) or CMOS (Complementary Metal Oxide Semiconductor), one or more optical lenses, lighting elements like LEDs (Light Emitting Diodes), and so on. The lighting elements can emit light with given colors, for example, white light, blue light, and so on.

One or more ultrasonic units 130 serve to output the ultrasound image data for the regions (hereinafter, referred to as 'submucosal regions') beneath the inner walls of the digestive organs or the surrounding organs around the digestive organs in real time or intermittently during the peroral endoscopic device 100 is moved along the digestive organs of the human body. The surrounding organs adjacent to the digestive organs include the pancreas, spleen, kidneys, liver, gallbladder, salivary glands, heart, lungs, and large intestine.

For example, the pancreas as one of the surrounding organs of the stomach is adjacent to the pylorus, pyloric antrum, or greater curvature of the stomach, and accordingly, the imaging units 120 perform the imaging for the inner walls of the stomach opposite to the pancreas within a very close region to discriminate whether the pancreas is normal or not. On the other hand, the ultrasonic units 130 perform the ultrasound imaging for the submucosal regions opposite to the pancreas to discriminate whether the pancreas is normal or not.

In this case, whether the surrounding organs of the patient, like the pancreas are normal can be checked simply, without having any ultrasound imaging using a probe or CT imaging for the region like the pancreas for which imaging is hard.

According to the present invention, the surface of the peroral endoscopic device 100 facing one or more ultrasonic units 130 may become flat. For example, if the surface of the peroral endoscopic device 100 facing one or more ultrasonic units 130 is the side surface of the peroral endoscopic device 100, the entire side surface is flattened or a portion (directly facing one or more ultrasonic units 130) of the entire side surface is flattened.

Further, one or more ultrasonic units 130 come into close contact with the facing surface of the peroral endoscopic device 100, so that they can perform the ultrasound imaging more accurately and clearly. In detail, the surface of the peroral endoscopic device 100 facing one or more ultrasonic units 130 becomes flat, so that the peroral endoscopic device 100 can come into close contact with the inner wall of the digestive organ, and accordingly, an air layer between the ultrasonic units 130 and the inner wall of the digestive organ does not exist, thereby achieving the clear ultrasound imaging.

The flat surface of the peroral endoscopic device 100 facing one or more ultrasonic units 130 or one or more ultrasonic transducers should have an outer surface of a body of the peroral endoscopic device 100. For example, only the outer surface of the peroral endoscopic device 100 is flattened and the inner surface facing the outer surface is curvedly formed. Otherwise, both of the inner surface and the outer surface facing the inner surface are flattened. It is important that the outer surface of the peroral endoscopic device 100 directly coming into close contact with the inner wall of the digestive organ has to be flattened, and in this case, an air layer between the ultrasonic units 130 and the inner wall of the digestive organ does not exist. Also, there is no need to make the inner surface of the peroral endoscopic device 100 on which the ultrasonic units 130 are located necessarily flattened.

If the ultrasonic units 130 are provided plurally on the peroral endoscopic device 100, further, they may be located on at least one of the front, back and side surfaces of the peroral endoscopic device 100. For example, if the peroral endoscopic device 100 has a shape of a cylinder whose circular surface in section is called the front, the opposite surface to the front is called the back, and the surface corresponding to the remaining column is called 'side surface', the ultrasonic units 130 are located only on the front, on both of the front and the side surface, on both of the front and the back, or all of the front, back and side surfaces and perform the ultrasound imaging simultaneously or with time differences. This is applied in the same manner as above even if the peroral endoscopic device 100 has the form of a capsule.

FIG.6 is a block diagram showing the ultrasonic unit of the peroral endoscopic device according to the present invention.

Referring to FIG.6, the ultrasonic unit 130 includes a plurality of HV (High Voltage) pulsers 131, an HV Tx/Rx switch 132, a transducer array 133, a plurality of pre-amps (pre-amplifiers) 134, a plurality of VGAs (Variable Gain Amplifiers) 135, a plurality of LPFs (Low Pass Filters) 136, and a plurality of ADCs (Analog/Digital Converters) 137.

The number of HV pulsers 131, the number of pre-amps 134, the number of VGAs 135, the number of LPFs 136, and the number of ADCs 137 are the same as the number of ultrasonic transducers located on the transducer array 133, and their operations are the same as those of the ultrasonic transducers.

If the control unit 160 receives the operation control signal that operates with an ultrasonic frequency to be activated from the monitoring device 400 or the magnetic force controller 200a, 200b, or 200c, it controls the plurality of HV pulsers 131 so as to generate a pulse corresponding to the ultrasonic frequency of the received operation control signal.

The plurality of HV pulsers 131 produces the pulse for generating the ultrasonic frequency of the received operation control signal and transmits the pulse to the HV Tx/Rx switch 132.

The HV Tx/Rx switch 132 is adapted to switch the pulse received from the plurality of HV pulsers 131 into the ultrasonic transducer corresponding to the pulse on the transducer array 133.

The transducer array 133 is a multi-transducer array on which the plurality of ultrasonic transducers is arrayed. The plurality of ultrasonic transducers is vibrated according to the pulses received from the HV Tx/Rx switch 132 to generate acoustic waves. Further, the transducer array 133 may have one transducer. This is because the peroral endoscopic device 100 according to the present invention is focused on single directional imaging, not on all directional imaging, and accordingly, it is possible to perform ultrasound imaging for the submucosal region through one transducer. As the single directional imaging, not all directional imaging is performed, therefore, the imaging results can be absolutely clear and errors caused by the changes in position of the ultrasonic images can be minimized, thereby performing the check-up more accurately.

The acoustic waves scanned along the scanning range like the inner wall of the stomach are reflected onto the inner wall and transmitted to the respective ultrasonic transducers.

The ultrasonic transducers of the transducer array 133 transmit the received reflected acoustic waves to the HV Tx/Rx switch 132, and the HV Tx/Rx switch 132 serves to switch the received reflected acoustic waves into the pre-amps 134 mapped on the respective ultrasonic transducers.

The pre-amps 134 serve to amplify the received reflected acoustic waves, and the VGAs 135 serve to variably amplify gains of the amplified acoustic waves.

The LPFs 136 serves to perform low pass for the acoustic waves whose gains are variably amplified, thereby removing noise from the acoustic waves, and the ADCs 137 serve to convert the acoustic waves from which the noise is removed into digital signals.

The control unit 160 serves to process the digital signals received from the ADCs 137 through a DSP (Digital Signal Processor) and controls the transceiving unit 110 to allow the processed digital signals to be transmitted to the receiver 300.

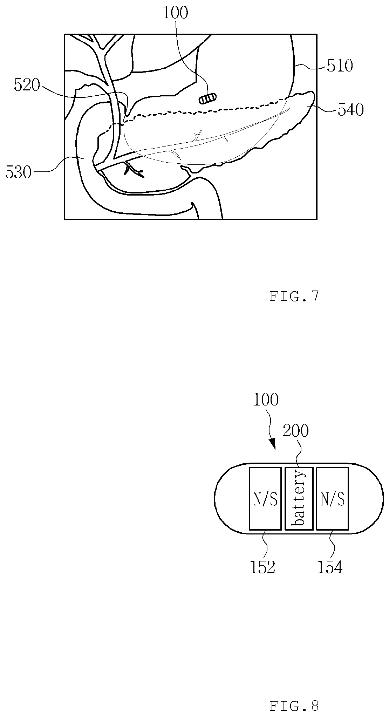

Referring to FIG.7, hereinafter, an operation of performing the ultrasonic imaging for the pancreas through the peroral endoscopic device 100 according to the present invention will be explained.

FIG.7 is a schematic view showing the stomach 510 and the pancreas 540 located around the stomach of the digestive organs of a human body.

Referring to FIG.7, the pancreas 540 is a thin and long organ having a length of about 15 cm and is located behind the stomach 510 in such a manner as to be connected to the duodenum 530 and adjacent to the spleen. The pancreas 540 is divided into a head, a body, and a tail. In detail, a portion of the pancreas 540 close to the duodenum 530 is the head, the middle portion thereof is the body, and the thinnest portion is the tail. Generally, diseases related to the pancreas 540 have different symptoms according to the portions of the pancreas 540 on which the diseases occur, and therefore, it is very important to detect positions where the diseases occur.

As shown in FIG.7, the pancreas 540 is close to the stomach 510 and the duodenum 530, and after the peroral endoscopic device 100 according to the present invention comes into close contact with the inner wall of the stomach 510 closest to the pancreas 540 through the magnetic force, the ultrasonic imaging for the head, body and tail of the pancreas 540 is performed with different frequencies from each other or with the same frequency as each other adequate for performing the ultrasonic imaging the pancreas 540.

At this time, the peroral endoscopic device 100 performs the imaging for the inner wall of the stomach 510 corresponding to the body or tail of the pancreas 540 at the antrum or greater curvature of the stomach 510 by means of the imaging units 120 and performs the ultrasonic imaging for the body of the pancreas 540 by means of the ultrasonic units 130.

If the pylorus 520 at which the stomach 510 and the duodenum 530 are connected is released by means of drugs, the peroral endoscopic device 100 is moved to the duodenum 530 through the external magnetic force of the magnetic force controller 200. Next, the peroral endoscopic device 100 performs the imaging for the inner wall of the duodenum 530 at a position corresponding to the head or tail of the pancreas 540 by means of the imaging units 120 and performs the ultrasonic imaging for the head or tail of the pancreas 540 by means of the ultrasonic units 130.

Referring again to FIG.5, the power unit 140 serves to supply power to the respective units 110, 120, 130, 150 and 160 of the peroral endoscopic device 100. The power unit 140 includes a chargeable battery or an exchangeable battery not rechargeable. A charging way of the chargeable battery is one selected from various wireless power charging, such as magnetic induction charging, magnetic resonance charging, and electromagnetic radiation charging. Further, the power unit 140 can turn the power on/off.

The magnetic unit 150 serves to adjust the posture and advancing direction of the peroral endoscopic device 100 in response to the external magnetic force and includes one or more permanent magnets. In detail, the magnetic unit 150 can perform the control in the posture and advancing direction of the peroral endoscopic device 100 when the peroral endoscopic device 100 is moved in position, rotates, and performs the imaging, through the magnetic force generated from the magnetic force controller 200.

Particularly, the magnetic unit 150 serves to allow the peroral endoscopic device 100 to come into close contact with the inner wall of the digestive organ by means of the interaction with the external magnetic force.

If the magnetic unit 150 includes two or more permanent magnets, the battery type power unit 140 is located between the two or more permanent magnets. Because the units having the biggest weights among the units of the peroral endoscopic device 100 are the magnetic unit 150 and the power unit 140, in this case, the arrangement where the permanent magnets of the magnetic unit 150 are located on both sides of the power unit 140 is advantageous in balancing the whole weight of the peroral endoscopic device 100. If the weight of the peroral endoscopic device 100 is seriously inclined toward any one side, it is very disadvantageous in that the magnetic force controller 200 controls the posture and advancing direction of the peroral endoscopic device 100. If the two or more permanent magnets are as far apart from each other as possible, further, the peroral endoscopic device 100 can be prevented from getting loose when it comes into close contact with the inner wall of the digestive organ through the external magnetic force of the magnetic force controller 200, thereby suppressing the formation of the air layer and thus giving advantages in view of the ultrasonic imaging.

FIG.8 is a concept view showing an example of an arrangement of two permanent magnets 152 and 154 and the power unit 140 in the peroral endoscopic device according to the present invention. Referring to FIG.8, the magnetic unit 150 includes the two permanent magnets 152 and 154, and the conductor type power unit 140 is located between the two permanent magnets 152 and 154. If the two permanent magnets 152 and 154 are arranged in the order of N/S poles and N/S poles, the magnetic unit 150 can have generally big N/S poles so that it can form a relatively strong magnetic force.

The control unit 160 controls the whole operation of the peroral endoscopic device 100. If the control unit 160 receives the operation control signals outputted from the monitoring device 400 or the magnetic force controller 200a, 200b, or 200c from the transceiving unit 110, the control unit 160 can process the received operation control signals to allow operations corresponding to the operation control signals to be executed.

For example, if the operation control signal through which the imaging units 120 and the ultrasonic units 130 simultaneously perform the imaging for the inner wall of the digestive organ is received, the control unit 160 controls the imaging units 120 and the ultrasonic units 130 so that the imaging for the inner wall and the submucosal region of the inner wall seen by the present imaging unit 120 and the present ultrasonic unit 130 is performed simultaneously or respectively through the present imaging unit 120 and the present ultrasonic unit 130.

If the magnetic force controller 200 controls the posture and advancing direction of the magnetic unit 150 so as to allow the peroral endoscopic device 100 to be moved to the inner wall of the stomach closest to the surrounding organs of the digestive organs, furthermore, the magnetic unit 150 responds to the control of the magnetic force controller 200 so that the peroral endoscopic device 100 is moved to the inner wall of the stomach closest to the surrounding organs. In this case, the control unit 160 can control the imaging units 120 to perform the imaging for the inner wall of the stomach closest to the surrounding organs. If the operator who monitors the monitoring device 400 commands the ultrasonic imaging in a state where it is found that the inner wall, for which the imaging is performed, is abnormal or not, the control unit 160 can control the ultrasonic units 130 to perform the ultrasonic imaging for the submucosal region of the inner wall coming into close contact with the peroral endoscopic device 100.

If the surrounding organ for close-up imaging is the pancreas, also, the operator can adjust the frequencies for imaging the head, tail and body of the pancreas. Accordingly, the control unit 160 can control the ultrasonic units 130 so that the pancreas imaging is performed in a state where the frequency for imaging the body of the pancreas is different from those for imaging the head and tail of the pancreas by means of the operation control signal received from the monitoring device 400 or the magnetic force controller 200a, 200b, or 200c. If the ultrasonic frequency becomes high, a resolution becomes increased, but a scan depth (or visible range) of the frequency becomes short. Contrarily, if the ultrasonic frequency becomes low, a resolution becomes decreased, but a scan depth (or visible range) of the frequency becomes long.

If the control unit 160 receives the image data from the imaging units 120 and the ultrasound data from the ultrasonic units 130, the control unit 160 produces the received image data and ultrasound data as the form of frames and controls the transceiving unit 110 to transmit the data to the receiver 300.

Furthermore, the control unit 160 can control the power unit 140 so that the power supplied to the imaging units 120 and the power supplied to the ultrasonic units 130 can be simultaneously or selectively turned on/off according to the operation control signal.

In addition, the control unit 160 can control the power unit 140 so that the power of the power unit 140 can be turned on/off according to the operation control signal.

Further, the control unit 160 can control the resolutions of the imaging units 120 or the ultrasonic units 130, that is, FPS (Frames Per Second) in a range of 2 to N.

Moreover, the control unit 160 transmits AGC (Automatic Gain Control) commands to the imaging units 120 and transmits the ultrasonic frequencies, voltages, and amplifier control commands to the ultrasonic units 130.

Hereinafter, the peroral endoscopic devices 100 according to first to fifth embodiments of the present invention will be explained with reference to FIGS.9 to 12B.

The peroral endoscopic device 100 as shown in FIGS.1 to 7 is one of the first to fifth peroral endoscopic devices 600 to 900 and 900a as will be explained with reference to FIGS.9 to 12B. Accordingly, the first to fifth peroral endoscopic devices 600 to 900 and 900a as shown in FIGS.9 to 12B include the units as shown in FIG.5, and a detailed explanation on the repeated units will be avoided for the brevity of the description.

Also, first to fifth imaging units, first to fifth ultrasonic units, first to fifth image sensors 610 to 910 and 910a, and first to fifth ultrasonic transducers 620 to 920 and 920a, as will be explained with reference to FIGS.9 to 12B, may become the imaging units 120, the ultrasonic units 130, sensors like CMOS of the imaging units 120, and the ultrasonic transducers arranged on the transducer array 133, as mentioned with reference to FIGS.5 and 6.

Further, portions of bodies 630 to 930 and 930a of the first to fifth peroral endoscopic devices 600 to 900 and 900a as shown in FIGS.9 to 12B are made of a material capable of performing ultrasonic detection.

First, FIG.9 is a schematic concept view showing the first peroral endoscopic device 600 according to the first embodiment of the present invention.

Referring to FIG.9, the first peroral endoscopic device 600 includes a first image sensor 610 of a first imaging unit and first ultrasonic transducers 620 of a first ultrasonic unit that are arranged serially on the same line as the first peroral endoscopic device 600 so as to perform imaging for the inner wall in the same direction as each other, that is, the same position as each other, in the digestive organ. In detail, the first image sensor 610 and the first ultrasonic transducers 620 are arranged serially on the same line as the first peroral endoscopic device 600.

At this time, the first image sensor 610 is located beside the outermost ultrasonic transducer among the first ultrasonic transducers 620.

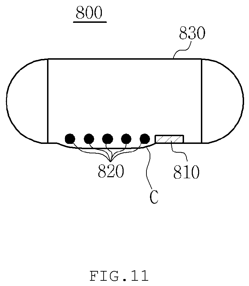

Further, FIG.9 shows one of an arrangement example where the plurality of first ultrasonic transducers 620 which is located in one first ultrasonic unit is arranged and an arrangement example where the plurality of first ultrasonic transducers 620 is arranged when one first ultrasonic transducer 620 is located in one first ultrasonic unit. This is the same as in the embodiments as shown in FIGS.10 and 11.

FIG.10 is a schematic concept view showing the second peroral endoscopic device 700 according to the second embodiment of the present invention.

The second peroral endoscopic device 700 as shown in FIG.10 is almost the same as the first peroral endoscopic device 600 as shown in FIG.9. However, a second image sensor 710 of the second peroral endoscopic device 700 is located between second ultrasonic transducers 720 so as to allow the angle of view of the second image sensor 710 to be overlapped as much as possible with the ultrasonic scanning range of the second ultrasonic transducers 720.

FIG.11 is a schematic concept view showing the third peroral endoscopic device 800 according to the third embodiment of the present invention.

The third peroral endoscopic device 800 as shown in FIG.11 is almost the same as the first peroral endoscopic device 600 as shown in FIG.9. However, the surface of the third peroral endoscopic device 800, on which third ultrasonic transducers 820 are located, that is, the surface of the third peroral endoscopic device 800 facing the third ultrasonic transducers 820, on which the ultrasonic waves are reflected, is formed as a curved surface having curvature C greater than 0. It is most desirable that the surface on which the third ultrasonic transducers 820 are located is flattened so as to prevent the ultrasonic waves scanned from the third ultrasonic transducers 820 from being passed through the air layer, but according to design factors, unavoidably, the curved surface may be formed on the outer surface of the third peroral endoscopic device 800. So as to allow the third peroral endoscopic device 800 to come into close contact with the inner wall of the digestive organ to the maximum, even in this case, the curved surface has to have positive curvature, and desirably, the curvature value is as small as possible.

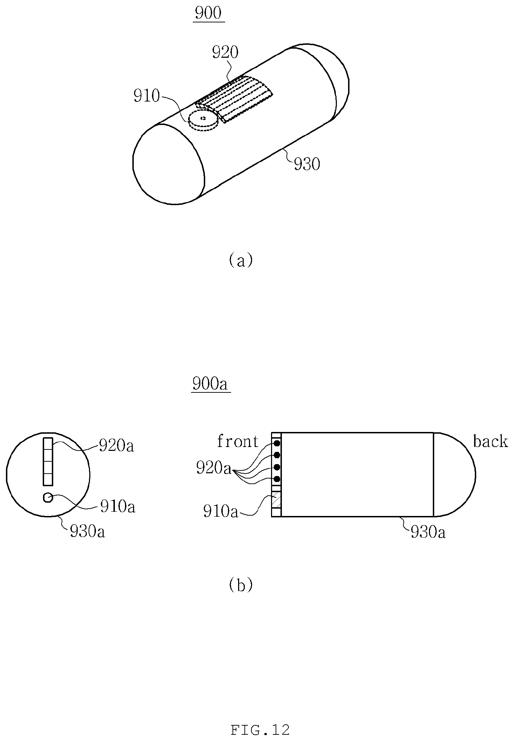

FIG.12A is a schematic concept view showing the fourth peroral endoscopic device 900 according to the fourth embodiment of the present invention.

Referring to FIG.12A, the fourth peroral endoscopic device 900 includes a fourth image sensor 910 and fourth ultrasonic transducers 920 arranged serially so as to perform imaging for the inner wall in the same direction as each other, that is, the same position as each other, in the digestive organ.

Particularly, the fourth image sensor 910 and the fourth ultrasonic transducers 920 are arranged in two rows and parallel with each other along the inner peripheral surface of the fourth peroral endoscopic device 900.

As shown in FIG.12A, the fourth image sensor 910 is spaced apart from the inner peripheral surface of the body of the fourth peroral endoscopic device 900 by a given distance, and even though not shown, it may come into close contact with the inner peripheral surface of the body of the fourth peroral endoscopic device 900. In this case, the shape or size of the fourth image sensor 910 is not particularly limited.

Also, the fourth ultrasonic transducers 920 are arranged along at least one arc of the fourth peroral endoscopic device 900 as a portion of the circumference thereof, that is, along the inner peripheral surface of the fourth peroral endoscopic device 900.

Even if not shown in FIG.12A, further, the fourth image sensor 910 and the fourth ultrasonic transducers 920 may be arranged on the entire peripheral surface of the fourth peroral endoscopic device 900.

FIG.12B is a schematic concept view showing the fifth peroral endoscopic device 900a according to the fifth embodiment of the present invention.

Referring to FIG.12B, the fifth peroral endoscopic device 900a has a shape of a cylinder, and a fifth image sensor 910a and fifth ultrasonic transducers 920a are arranged on the same line as each other on any one surface of the front and back surfaces of the fifth peroral endoscopic device 900a.

As shown in FIG.12B, the surface of the fifth peroral endoscopic device 900a, on which the fifth ultrasonic transducers 920a are located, that is, which faces the fifth ultrasonic transducers 920a, is flattened, and accordingly, the fifth ultrasonic transducers 920a come into close contact with the flattened surface of the fifth peroral endoscopic device 900a.

The first to fifth peroral endoscopic devices 600 to 900 and 900a as shown in FIGS. 9 to 12B are provided with their respective first to fifth ultrasonic transducers, but of course, they may be provided with one ultrasonic transducer.

If the ultrasonic sensors are applied to the sides or front surfaces of the respective bodies 630, 730, 830, and 930a of the first to third peroral endoscopic devices 600, 700 and 800 and the fifth peroral endoscopic device 900a as shown in FIGS.9, 10, 11, and 12B, their sides or front surfaces are flattened, so that they can uniformly come into close contact with the inner wall of the digestive organ.

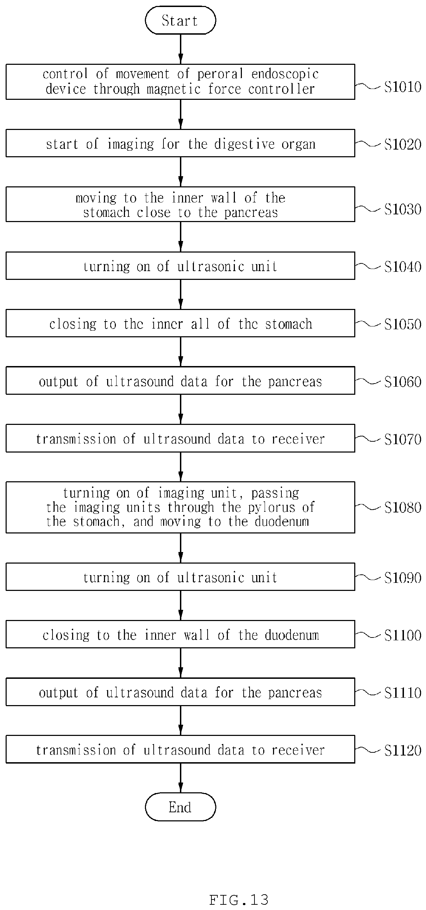

FIG.13 is a flowchart showing an endoscopic method of the pancreas through the peroral endoscopic device 100 according to the present invention.

Referring to FIG.13, if the peroral endoscopic device 100 enters a patient's digestive organ, the operator controls the movement of the peroral endoscopic device 100 through the magnetic force controller 200 placed on the patient's abdominal region (Step S1010).

The peroral endoscopic device 100 activates the imaging units 120 through the operation control signal outputted from the monitoring device 400 or the magnetic force controller 200a, 200b, or 200c to perform imaging for the digestive organ (Step S1020).

The peroral endoscopic device 100 is moved to the inner wall of the stomach closest to the pancreas through the external magnetic force of the magnetic force controller 200 (Step S1030). In detail, while the operator is monitoring the images obtained from the imaging units 120 through the monitoring device 400, the position closest to the pancreas is determined by the operator, and the magnetic force controller 200 is controlled to allow the peroral endoscopic device 100 to be moved to the determined position.

The peroral endoscopic device 100, which is moved to the inner wall of the stomach closest to the pancreas, turns the imaging units 120 off and turns the ultrasonic units 130 on by means of the operation control signal if necessary (Step S1040) .

After the peroral endoscopic device 100 comes into close contact with the inner wall of the stomach to the maximum through the external magnetic force of the magnetic force controller 200, 200a, 200b, or 200c, it controls the ultrasonic units 130 to generate frequencies corresponding to the ultrasonic frequencies determined by the monitoring device 400 or the magnetic force controller 200a, 200b, or 200c (Step S1050).

Next, the ultrasonic units 130 of the peroral endoscopic device 100 generate acoustic waves corresponding to the ultrasonic frequencies, receive the acoustic waves reflected onto the inner wall, perform the signal processing like gain amplification, digital conversion, and so on, and output ultrasonic data (Step S1060). As a result, the ultrasonic data corresponding to a portion of the pancreas is outputted.

The peroral endoscopic device 100 transmits the ultrasonic data outputted at the step S1060 to the receiver 300 (Step S1070).

If the ultrasonic imaging for the portion of the pancreas is finished through the step S1070, it is checked whether the pylorus of the stomach is released by means of drugs injected before the step S1010.

If it is checked that the pylorus is released to a degree where the peroral endoscopic device 100 is movable, the peroral endoscopic device 100 is passed through the pylorus of the stomach through the external magnetic force of the magnetic force controller 200 and is then moved to the duodenum close to the head of the pancreas (Step S1080). At or after the step S1070, the ultrasonic units 130 are turned off, and the imaging units 120 are turned on so that lighting elements emit light.

Next, the peroral endoscopic device 100 turns on the ultrasonic units 130 on the inner wall of the duodenum close to the head of the pancreas through the operation control signal (Step S1090). At the steps S1040 and S1090, the imaging units 120 are kept turned on or are turned off, which is selectively applied in consideration on the surrounding situations. The surrounding situations include a residual amount of the battery and states of the inner/outer walls of the stomach, pancreas, and duodenum.

After the peroral endoscopic device 100 comes into close contact with the inner wall of the duodenum through the external magnetic force of the magnetic force controller 200, 200a, 200b, or 200c, it controls the ultrasonic units 130 to generate the frequencies corresponding to the ultrasonic frequencies determined by the monitoring device 400 or the magnetic force controller 200a, 200b, or 200c (Step S1100).

The ultrasonic units 130 of the peroral endoscopic device 100 perform the imaging for the pancreas through the acoustic waves corresponding to the ultrasonic frequencies and output ultrasonic data (Step S1110).

The peroral endoscopic device 100 transmits the ultrasonic data outputted at the step S1110 to the receiver 300 (Step S1120).

The monitoring device 400 analyzes and processes the image data and the ultrasonic data received from the receiver 300 and displays the processed signal on the screen.

On the other hand, the endoscopic method for the pancreas through the peroral endoscopic device 100 according to the present invention is implemented typologically by means of programs of commands for implementing the endoscopic method, and accordingly, the endoscopic method can be recorded in a computer readable recording medium, which will be easily understood by those having the ordinary skill in this art.

In detail, the endoscopic method for the pancreas through the peroral endoscopic device 100 according to the present invention may be implemented in the form of a program instruction that can be performed through various computers, and may be recorded in a computer readable recording medium. The computer readable medium may include a program command, a data file, a data structure, and the like independently or in combination. The computer readable recording medium may include a magnetic medium such as a hard disc, a floppy disc, and a magnetic tape, an optical recording medium such as a Compact Disc Read Only Memory (CD-ROM) and a Digital Versatile Disc (DVD), and a hardware device specifically configured to store and execute program instructions, such as a Read Only Memory (ROM), a Random Access Memory (RAM), a flash memory, and a USB memory.

Accordingly, the present invention also provides the program recorded in the computer readably recording medium that is implemented on the computer controlling the peroral endoscopic system so as to implement the endoscopic method for the pancreas through the peroral endoscopic device 100 according to the present invention.

As described above, the peroral endoscopic device according to the present invention is controlled in a moving direction, a moving speed, and an imaging direction by means of the magnetic force, thereby achieving accurate monitoring and diagnosis for various digestive organs such as the relatively large stomach as well as the small and large intestines.

Through the small ultrasonic elements, in addition, the peroral endoscopic device according to the present invention can more accurately diagnose diseases hard to be accurately diagnosed with the conventional optical peroral endoscope.

Further, the peroral endoscopic device according to the present invention is provided with both of the magnets and the ultrasonic elements, so that it can be miniaturized and made at a low cost, thereby carefully performing the imaging for the inner wall of the digestive organ as well as the submucosal region of the inner wall.

Furthermore, the peroral endoscopic device according to the present invention comes into close contact with the inner wall of the stomach through the magnetic force to perform the ultrasonic imaging for the submucosal region of the stomach and the surrounding organs around the stomach more obviously, so that it is unnecessary to perform no additional CT imaging or the endoscopic ultrasonography using a probe, thereby improving the conveniences of the patient.

The present invention is usefully adopted as the peroral endoscopic device that is capable of monitoring the digestive organs and the surrounding organs around the digestive organs.

While the present invention has been described with reference to the particular illustrative embodiments, it is not to be restricted by the embodiments but only by the appended claims. It is to be appreciated that those skilled in the art can change or modify the embodiments without departing from the scope and spirit of the present invention.

## Claims

1. A peroral endoscopic device capable of being swallowed, comprising:
one or more imaging units moving to the digestive organs of the human body, performing imaging for the digestive organs, and outputting image data for the digestive organs;
one or more ultrasonic units for outputting ultrasound data for submucosal regions beneath the inner walls of the digestive organs and the surrounding organs around the digestive organs;
a magnetic unit for adjusting a position, a posture and an advancing direction of the peroral endoscopic device in response to an external magnetic force;
a transceiving unit for transmitting the image data and the ultrasound data to an external device or for receiving an external control signal;
a control unit for controlling one or more imaging units and one or more ultrasonic units so as to perform the imaging for the digestive organs and the submucosal regions simultaneously or individually; and
a power unit for supplying power to one or more imaging units, one or more ultrasonic units, the magnetic unit, the transceiving unit, and the control unit.

2. The peroral endoscopic device according to claim 1, wherein the external magnetic force is generated from an external magnetic force controller for operating the magnetic unit, and the magnetic force controller comes into close contact with or is close to an upper body of a patient to apply the external magnetic force to the magnetic unit.

3. The peroral endoscopic device according to claim 2, wherein the magnetic force controller controls the magnetic unit to allow the peroral endoscopic device to be moved to the inner wall of the stomach closest to the surrounding organs, and if the peroral endoscopic device is moved to the inner wall of the stomach closest to the surrounding organs, the control unit controls one or more imaging units to perform the imaging for the inner wall of the stomach closest to the surrounding organs and one or more ultrasonic units to perform the imaging for the submucosal region of the inner wall of the stomach closest to the surrounding organs.

4. The peroral endoscopic device according to claim 1, wherein if the surrounding organ is the pancreas, the control unit controls one or more ultrasonic units so that pancreas imaging is performed in a state where a frequency for imaging the center of the pancreas is different from frequencies for imaging the head and tail of the pancreas.

5. The peroral endoscopic device according to claim 1, wherein an image sensor of one or more imaging units and one or more ultrasonic transducers of one or more ultrasonic units are arranged serially on the same line as the peroral endoscopic device so as to perform the imaging for the inner wall of the digestive organ in the same direction as each other.

6. The peroral endoscopic device according to claim 5, wherein if the ultrasonic transducers are two or more, the image sensor of one or more imaging units is located between the ultrasonic transducers so as to allow the angle of view of the image sensor to be overlapped with the ultrasonic scanning range of the ultrasonic transducers.

7. The peroral endoscopic device according to claim 1, wherein an image sensor of one or more imaging units and one or more ultrasonic transducers of one or more ultrasonic units are arranged in two rows and parallel with each other along the inner peripheral surface of the peroral endoscopic device so as to perform the imaging for the inner wall of the digestive organ in the same direction as each other.

8. The peroral endoscopic device according to claim 7, wherein one or more ultrasonic transducers of one or more ultrasonic units are arranged along at least one arc of the peroral endoscopic device as a portion of the circumference thereof.

9. The peroral endoscopic device according to claim 1, wherein the magnetic unit is adapted to allow the peroral endoscopic device to come into close contact with the inner wall of the digestive organ through the interaction with the external magnetic force.

10. The peroral endoscopic device according to claim 1, wherein one or more ultrasonic units come into close contact with the surface of the peroral endoscopic device that faces the ultrasonic units.

11. The peroral endoscopic device according to claim 10, wherein the surface of the peroral endoscopic device facing one or more ultrasonic units is flattened.

12. The peroral endoscopic device according to claim 11, wherein if the ultrasonic units are two or more, the ultrasonic units are arranged on at least one surface of the front, back, and side surfaces of the peroral endoscopic device.

13. The peroral endoscopic device according to claim 11, wherein the surface of a body of the peroral endoscopic device facing one or more ultrasonic transducers of one or more ultrasonic units is flattened, so that one or more ultrasonic transducers come into close contact with the flattened surface.

14. The peroral endoscopic device according to claim 1, wherein the magnetic unit comprises:
a first permanent magnet; and
a second permanent magnet, and
the power unit as a conductor is located between the first permanent magnet and the second permanent magnet.

15. The peroral endoscopic device according to claim 14, wherein the magnetic unit is adapted to allow the peroral endoscopic device to come into close contact with the inner wall of the digestive organ through the interaction with the external magnetic force.

16. The peroral endoscopic apparatus of claim 1, wherein the magnetic unit includes:
a first permanent magnet; and
a second permanent magnet,
wherein the power supply unit as a conductor is provided between the first permanent magnet and the second permanent magnet.

17. The peroral endoscopic apparatus of claim 16, wherein the magnetic unit allows the peroral endoscopic apparatus to be in close contact with the inner wall of the digestive system by interaction with the external magnetic force.
